# EUROPEAN PATENT APPLICATION

(11) **EP 2 248 417 A2**
(43) Date of publication of application: **10.11.2010**
(21) Application number: 10162323.9
(22) Date of filing: 07.05.2010
(51) Int. Cl.: A01K 67/033

(54) **Process for rearing a species of indigenous parasitoid and use thereof for controlling lepidopteran crop pest**

(30) Priority: 08.05.2009 ES 200901183
(71) Applicant: Agrobio S.L., 04745 El Viso (La Mojonera) - Almeria (ES)
(72) Inventor: Vila Rifà, Enrique, 04745 El Viso (La Mojonera)- Almería (ES); Garcia, Tomás Cabello, 04740 Roquetas de Mar (Almería) (ES)
(74) Representative: De Hoop, Eric

(57) **Abstract**

The invention relates to a process for rearing the parasitoid *Trichogramma achaeae,* the use of *Trichogramma achaeae* for biological pest control in commercial crops, and a composition comprising *Trichogramma achaeae.* The invention provides a process for rearing *Trichogramma achaeae* on the moth *Ephestia kuehniella* or *Sitotroga cerealella* as factitious host. Further the invention provides the use of *Trichogramma achaeae* for biological pest control in commercial crops, wherein *Trichogramma achaeae* is reared on the moth *Ephestia kuehniella* or *Sitotroga cerealella* as factitious host. Also the invention provides a composition comprising: a viable population of *Trichogramma achaeae,* and the moth *Ephestia kuehniella* or *Sitotroga cerealella* as factitious host.

## Description

In general, the present invention relates to a process for rearing and using a species of indigenous parasitoid for biological control of lepidopteran crop pests.

The introduction of biological programs which use arthropod species as control agents has been arousing, for the second half of the 20th century, a continuous interest among producers of a wide range of crops, particularly in warm geographical regions, such as the Mediterranean basin. So much so that this practice has grown from amateur led schemes by individual growers to large commercial developments where international companies produce beneficial agents year round in large quantities, which are supplied to countries on a worldwide basis.

The benefits accruing to growers and consumers resulting from a corresponding decrease in the use of chemical pesticides is obvious and very considerable. So that the practice of biological control is growing steadily, year by year, as government health and safety regulators, wholesale companies and their retail customers increase their demand for chemically clean produce. This demand is also clearly stated by the agricultural policy of the European Community since 1992, which aim is to decrease the pesticides use and to establish Integrated Pest Management of crops, using biological control as the main tool.

In southeast Spain, particularly during the last years, a fast growing of the crop surface where integrated pest management (IPM) programs are used has occurred. Specifically in Almeria, which is one of the largest vegetable producing areas in Europe, the surface where IPM is practiced has increased from 1,400 hectares in the 2006/07 season to 19,785 hectares in 2009/10. This increase of IPM programs means an unprecedented challenge and it is especially necessary for the vegetable sector to keep on competitive, considering the market evolution. Nevertheless, IPM application has not carried out equally on different crops. For example, biological control is still applied in a low percentage of tomato greenhouse crops. Moreover, for this type of crop an exotic pest, the lepidopteran *Tuta absoluta* (Meyrick) (Lep.: Gelechiidae) has recently appeared, which is causing serious problems and for which, to date, there is no satisfactory solution.

*Tuta absoluta* has spread from Central America to most of South America and it is considered one of the main tomato and other solanaceae pests (EPPO, 2005). Its chemical control is difficult for different reasons. Firstly, owing to the biology of the lepidopteran *Tuta absoluta*, since larvae are located in the leaves or inside the fruits (Branco and Franca, 1993; Urbaneja et al., 2007), which causes the necessity of an intensive use of chemical control. Secondly, due to the appearance of resistance to the active compounds used until now (EPPO, 2005). For this reason, biological control of the pest species using natural enemies, has been developed and applied by several countries of South America.

*Tuta absoluta* was introduced in Castellón, Spain, in 2006. It extended from Valencian Community to Murcia and Ibiza (Urbaneja et al., 2007; EPPO, 2008) and the first attacks on greenhouse crops in Almeria were observed in 2008. The pest has developed big harshness in the last seasons on tomato crops in greenhouses of the Murcia area (Lucas et al., 2009). From Spain it is spreading quickly over other European countries, like Italy, France, Holland, UK, Malta, Switzerland, Germany, Cyprus, Bulgaria, Lithuania and Russia, where it is also causing damages (http://archives.eppo.org/EPPOReporting/REporting_Archives .htm).

In Spain various indigenious predators have been assessed to control *Tuta absoluta*, for example by using the hemipteran predators *Nesidiocoris tenuis* (Reuter) and *Macrolophus pygmaeus* (Rambur) (Urbaneja et al., 2008), but neither of these predators can supply a satisfactory control of the pest. The natural enemies which are commercially available to control this pest come from South America and they are exotic in Europe. For that reason, its introduction in Spain would become a big environmental risk.

Therefore, there is a big need to find a solution for this quarantine pest, which can put tomato exports at risk. It is especially necessary the possibility of having an indigenous species, then without environmental risks, and which can carry out a suitable control. Moreover, this species must be relatively easy to rear on a large scale, so that it can be available in the necessary amounts and in affordable price for farmers.

Qualified people of the applicant company have assessed different indigenous parasitoid species which could be easily reared on a large scale (around 50 species). Among them, the indigenous species *T. achaeae* has been selected. This species presents a wide world distribution. *T. achaeae* has been described in Asia (China, India and Russia), Europe (France, Russia), Africa (Cape Verde) and the New World (Argentina, Barbados, Chile, Trinidad and Tobago, USA.) and recently in Spain. *T. achaeae* is an egg parasitoid species of 26 lepidopteran species belonging to 10 families: Gelechiidae, Geometridae, Noctuidae, Notodontidae, Oecophoridae, Pieridae, Pyralidae, Sphingidae, Tortricidae and Yponomeutidae (Polaszek, data not released). However, *T. achaeae* had never been described before as a parasitoid of *Tuta absoluta* or any other species of this genera. In Europe, where *Tuta absoluta* was not present, *Trichogramma* achaeae had never been in contact with this moth pest in the nature.

Therefore a first embodiment of the invention is a new rearing process for the commercial mass rearing of the indigenous parasitoid species *Trichogramma* achaeae Nagaraja & Nagarkatti (Hym.: Trichogrammatidae).

In a second embodiment, the invention concerns the use of the indigenous parasitoid species *Trichogramma* achaeae for controlling lepidopteran crop pest. Thus, another embodiment of the invention is the use of the indigenous parasitoid species *Trichogramma* achaeae reared in accordance with the first aspect of the invention for controlling lepidopteran crop pest.

The introduction of the commercial use of this species will increase the range of biological control agents available to control lepidopterans. It offers solutions for determined pests for which, to date, satisfactory control methods had not been found. Besides, it generates at the same time competition among the producers for other pests that already have natural enemies, thus reducing the costs for farmers. Moreover, at present, no other species of *Trichogramma* have been broadly used against pest in horticultural protected crops in Europe, most probably because the species largely used in open field crops, like *T. brassicae*, which offers a satisfactory control of the European Corn Borer, *Ostrinia nubilalis* Hübner (Lepidoptera, Pyralidae), in maize, are not showing a good adaptation to greenhouses conditions. The commercial mass rearing of *T. achaeae,* that has showed a very good behavior in greenhouses, can provide, for the first time, cost effective biological control programs against Lepidoptera pests in protected crops.

An important aspect of the biological control strategies where entomophagous (parasitoids and predators) are used is determined by the biological features of the colonies used. Thus, on many occasions it has been proved that the application of the concept of taxonomical species (only based in morphological characteristics) is insufficient to obtain an effective biological control agent (Schlinger and Doutt, 1984; Waterhouse, 1998; Huber et al., 2001; Waterhouse and Sands, 2001; Driesche et al., 2007). In this regard, biological control effectiveness is determined, among other parameters, by the wide genetic variability for a certain species. Therefore, an adaptation to the local geographical conditions is occurred, which leads to multiple and diverse characteristics as regards biology, ecology or host/prey ratio. In this way, due to such variability, effectiveness is also very different when the agent is applied as a biological control agent.

In a first embodiment of the invention, *T. achaeae* is reared over the factitious hosts *Ephestia kuheniella* Zell. (Lepidoptera: Pyralidae) or *Sitotroga cerealella* Olivier (Lepidoptera: Gelechidae). A factitious host is a species which inhabits a different natural habitat than the parasitoid, but nevertheless one or more life stages of the factitious host (the eggs in such case) are suitable for the development of at least one life stage of the parasitoid (in this case the egg, larvae and pupa). *E. kuheniella* and *S. cerealella* are pests of farinaceous products, and are very common in stored food products, being never described in plants or crops.

In a preferred embodiment of the application, the parasitoid *Trichogramma* achaeae is used as a composition of at least two different subspecies of *T. achaeae,* preferably three different subspecies. These subspecies have different biological characteristics and different parasitic activities for eggs of *Tuta absoluta*. The mixture of two or more of these subspecies has a parasitic activity which is significantly bigger than the mixture of individual genotypes. This is probably due to the mixture and the selective mating of adults of each subspecies, which is carried out one generation prior to its use, under field conditions, in order to be applied as biological control agents. Thus, a hybrid vigor is obtained. The applicants have collected individuals of three different populations of *T. achaeae* from different Canary islands, all of them on tomato plants that were grown in places with volcanic soil, where the temperatures were rather warm (up to 40°C during the middle of the day). Then, in a further embodiment of this invention the populations were collected from areas with subtropical climate during summer period, with temperatures very similar to the ones that occur in protected Mediterranean crops. The local conditions where the populations were collected, may condition the behavior and biology of the individuals, and most probably the very good adaptation to greenhouse crops that has been later stated when experimentally applied by the applicants against horticultural moth pests, like *Tuta absoluta*, *Chrysodeixis chalcites* and *Heliothis armigera*.

*T. achaeae* fulfills all the requirements to be commercially successful, because it is a simple, reliable and cheap system which provides big sustainable populations to be used as parasitoid of crop pests. Moreover it is a fast and cheap rearing species.

The populations of the factitious hosts *E. kuheniella* and *S. cerealella* are used in the form of UV sterilized eggs. The UV system ensures that all the populations of the factitious host are dead without denaturing the protein content, then allowing a good development of the parasitoid. For the rearing process of *Trichogramma* achaeae, a single layer of eggs of the factitious host are offered to the adults of the parasitoid in a cage or container. Once the eggs are parasitized, they are isolated on different containers and a new batch of eggs are offered to the adults. This is repeated until the dead of the adults. All the parasitized eggs are preserved in a suitable container for the adequate development of the parasitoid, guaranteeing the exchange of oxygen and enabling the exit of the adult parasitoids, emerged from the eggs of its factitious host. Many different shapes and sizes of containers have been used to rear species of parasitoids like *Trichogramma* species, according to the needed quantity of mass production. The use of these factitious hosts enables to multiply the populations of *T. achaeae* by 35 in 10 days. Moreover, the physical requirements to succeed in the rearing are very reasonable, preferably a temperature around 20-25°C, preferably 22°C, and 65-85% of relative humidity. Referring to the second aspect of the invention, this refers to the use of the parasitoid *T. achaeae*, produced in the aforementioned system according to the described rearing process, for biological pest control in commercial crops. In a specially preferential embodiment of the invention, the crop pest is selected from gelechii lepidopterans, such as the exotic South American tomato moth *Tuta absoluta*, with which the parasitoid has no previous contact in the wild, and noctuidae such as *Helicoverpa armigera* (= *Heliothis armigera*) (Hübner) (Lepidoptera: Noctuidae) and *Chrysodeixis chalcites* (Esper, 1789) (Lepidoptera: Noctuidae).

Lab and field tests about the percentage of reduction of damages caused by *Tuta absoluta* have been carried out in tomato crops. These tests prove effectivities of up to 97% of reduction of damages (see Example 1). Such results prove that the populations of *T. achaeae* obtained in accordance to the rearing process described in this invention involve substantial advantages against other species known for being natural enemies of *Tuta absoluta* in other continents. On the one hand, *T. achaeae* is an indigenous species, and, thus, without environmental risks. On the other hand, greater effectivities than those described by other authors in South America with other species have been reached.

According to the second embodiment of the invention, *T. achaeae* is introduced in greenhouse and outdoors tomato crops to control the moth pest *Tuta absoluta*, using cardboard cards which are hung on the plants, to be distributed in the crops. From 50 to 400 cardboard dispensers per hectare with up to 10,000 pupae of *T. achaeae* each one are distributed periodically on the greenhouses. A successful control is reached with weekly or bimonthly introduction of between 250,000 and 1,000,000 individuals during the flying period of adults of *Tuta absoluta*. Likewise, *T. achaeae* is introduced in greenhouse and outdoors tomato crops to control the moth pest *Tuta absoluta* adhered to an inert material which acts as carrier. It can be distributed in the crop with direct sprinkling.

In another way of doing the invention, and according with this second embodiment of it, *T. achaeae* is used to control the moth pest *T. absoluta* in other vegetables different to tomatoes where this pest is also causing damages, for example potatoes and egg plants, among others, using the same methodology described for tomatoes.

According to another embodiment of the invention, *T. achaeae* is used to control other species of lepidopteran crop pests, such as the case of the moth *Chrysodeixis chalcites* on banana crops in the Canary Islands and Heli*coverpa armigera* on tomato crops (in greenhouse as well as open field crops) and on cotton crops. In recent years the tomato looper, *Chrysodeixis chalcites*, has become one of the most important noctuidae pests in banana tree crops in the Canarie, showing its control diverse difficulties due to the low level of the effectiveness of chemical or biological insecticides and the use of inadequate control strategies. Likewise, an increase of the damages caused by this and other noctuidae pest, such as *H. armigera*, has been verified in different vegetable crops due to the slim effectiveness of chemical control because of the pest resistance to most of the available active compounds.

Thus, according to such embodiments of the invention, the subespecies of *T. achaeae* obtained by the aforementioned process, will be used in multiple biological control as biological control weapon against some of the most serious lepidopteran pests, namely the species of *Tuta absoluta, Chrysodeixis chalcites* and *Helicoverpa armigera*. Preferably, the commercial crop is selected from among protected vegetable crops of glass or plastic greenhouses, or under mesh and also outdoors crops. For example in tomato, egg plant, strawberry raspberry, cotton plant and banana tree crops.

The invention also provides a composition comprising: a viable population of *Trichogramma* achaeae, and the moth *Ephestia kuehniella* or *Sitotroga cerealella* as factitious host.

### Examples: Technical tests for assessing the potential control of Tuta absoluta with the indigenous parasitoid T. achaeae in a greenhouse tomato crop.

The aim of these tests was assessing the effectiveness of *T. achaeae* to control *T. absoluta* on greenhouse tomato crops. For this reason lab, semi-field, and field tests have been carried out in summer-autumn period (Example 1) and in winter-spring period (Examples 2 and 3).

### Example 1: Lab and semi-field tests in summer-autumn tomato crops

### Materials and Methods

### Insect rearing

The specimens of *T. absoluta* used in all the tests were obtained from populations reared under laboratory on tomato leaves. In turn, the rearing of *T. achaea*, was carried out using *Ephestia kuehniella* Zeller as factitious host, in climatic rooms (25±1° C, 60-80 % RH and 16:8 hours L:D). For this rearing, a single layer of eggs of the factitious host, previously sterilized with UV, were attached to a piece of paper, and the eggs were offered to the adults of the parasitoid in plastic bottles (200 mm large x 90 mm diameter). The bottle was closed with a lip which had a hole sealed with filter paper, for ventilation. All the parasitized eggs were preserved in the plastic container until the exit of the adult parasitoids, emerged from the eggs of its factitious host.

### Lab tests

A preliminary test under laboratory conditions (25±1° C, 60-80 % RH and 16:8 hours L:D) was carried out to evaluate acceptance and parasitation of *T. absoluta* eggs by females of the parasitoid. Methodology was adapted from Brotodjojo & Walter (2006), with the exception that 10 eggs of *T. absoluta* (less than 24 hours aged) were offered to each female. 10 repetitions were conducted. All the parasitized eggs were developed until adult emergence.

### Semi-field tests in commercial greenhouses

To evaluate the efficacy of *T. achaeae* a test was conducted in a commercial greenhouse of Almeria (Spain), between August the 27th and September the 22nd of 2009. Eight cages of 8 m² containing cherry tomato plants transplanted on pots (20 plants per cage) were set in the greenhouse. The plants were infested with adults of *T. absoluta* when reaching 1 m of height (release of 4 adults per plant). A total of 7 releases of *T. achaeae* (30 adults per plant) were carried out every 3 or 4 days in 4 cages; the other 4 cages were left as a control group. The number of larvae of *T. absoluta*, leaves mines and damaged fruits were counted after 27 days. Data was analyzed using one-way ANOVA and the means were compared with Least Significant Difference (LSD), using the software SPSS v. 15 (SPSS, 2006).

### Results

### Lab tests

We found that host eggs were well accepted by the females of *T. achaeae,* since 100% of the eggs received a lay of the parasitoid. The 83.3% of the eggs turned black (apparent parasitism; when the parasitoid has reached the pupa phase), the remainder of them did not do, getting collapsed in the end. This can be due to the small size of the host egg (length = 372.46 ± 9.24 µ and width = 233.98 ± 6.39 µ; n=25).

### Greenhouse tests

In Table 1 the number of larvae of *T. absoluta*, leaf mines and damaged fruits per plant, in greenhouse tomatoes, 27 days after initiating the test, are indicated. During this period, temperatures oscillated between the limits of 38.6°C (absolute maximum temperature) and 22.2°C (absolute minimum temperature) with a mean value of 29.0°C. In the ANOVA highly significant effects of the treatment (P < 0.01) in the number of larvae and leaf mines have been found. The effect has also been significant (P < 0.05) in the number of damaged fruits. The number of larvae of the pest species is almost 12 times lower in the plots where the *T. achaeae* were released compared to the control group, which represent an efficacy of 91.74 %.

**Table 1**

| **Numbers of larvae of *Tuta absoluta* and damages in greenhouse tomato crops according to the treatment, in summer conditions** | | |
|---|---|---|
| (The averages with different letters are statistically different) | | |
| **Number per plant** | **Control group** | **Group with releases of *T.achaeae*** |
| Larvae of *T. absoluta* | 5.0a | 0,4b |
| Leaf mines of *T. absoluta* | 23.5^{a} | 5,8b |
| Damaged fruits | 0.9a | 0,1b |

This data has been published in an international journal, see reference Cabello et al., 2009, after this application was first submitted to the Spanish Patent Office.

### Example 2: Field tests in winter-spring tomato crops

### Materials and Methods

### Insect rearing

The individuals of *Tuta absoluta* and *Trichogramma* achaeae came from the same rearing conditions previously described in the Example 1.

### Greenhouse test

We carried out the test in a 400 square meter experimental greenhouse in the Experimental Station of the Cajamar Foundation 'Las Palmerillas', in Almeria, in winter and spring of 2009. We planted a tomato crop with a density of 2 plants per square meter. The greenhouse was divided in two plots, setting a mesh in the center. Two weeks after the transplant, the predator *Nesidiocoris tenuis* was introduced in a ratio of 0.5 individuals per m² (usual dose of releases of this natural enemy in spring tomato crops in Almeria). We also released in the whole crop 4 adults of *T. absoluta* per plant 3 weeks after the crop trasplant. A plot was used as control group, without releases of parasitoids. In the other plot *T. achaeae* were released, starting the day after the introduction of *T. absoluta*. 60 individuals of *T. achaeae* were introduced two or three times per week, all the weeks in which active adults of the pest were observed (captures in yellow glue traps). The number of eggs of *T. absoluta*, the number of leaf mines with or without larvae and the damaged fruits were counted every week in a total of 20 plants per plot. Samples of leaves with eggs of *T. absoluta* were collected every week, and the number of eggs which were parasitized were examined in the lab.

### Results

The results were counted during the first 7 weeks of growing, period in which we can already appreciate clear results of the effectiveness of the parasitoid *T. achaeae*. Within a wide range of temperatures, 42°C as maximum temperature and 6°C as minimum, a parasitism percentage of 97.6% was obtained, by means of releases of the parasitoid *T. achaeae*. The reduction of damages in leaves per plant was a 92.5% compared to the control group, where only the predator *N. tenuis* was introduced to control the pest.

### Example 3: Semi-field tests in tomato crops to assess the compatibility of use of T. achaeae with the predator N.tenuis in winter-spring tomato crops

### Materials and methods

### Insect rearing

The individuals of *T. absoluta* and *T. achaeae* came from the same rearing conditions previously described in the Example 1.

### Greenhouse test

We carried out the test in a commercial greenhouse of Almeria (Spain), during the spring of 2009. 8 m² cages were set up in the greenhouse. They contained cherry tomato plants transplanted in pots (20 plants per cage). When the plants reached the height of 1 m, they were infested with adults of *T. absoluta* (release of 4 adults per plant). In 2 cages, only 20 individuals of *Nesidiocoris tenuis* (1 individual per plant) were released. In other 2 cages, a total of 7 releases of 60 individuals of *T. achaeae* per plant were carried out, in three consecutive weeks (2-3 releases per week). In 2 cages both natural enemies, *N. tenuis* and *T. achaeae*, were introduced using the same rates described in the other 2 treatments. The last 2 cages were left as a control group, without natural enemies. The number of larvae of *T. absoluta*, leaf mines and damaged fruits were counted in 6 plants per cage every week.

### Results

A high level of parasitism (95,3%) was reached in the cages where releases of the parasitoid *T. achaeae* were done. A sharp decrease of damages occurred, compared to treatments with only the predator *N. tenuis* or without natural enemies.

The results about the effectivity of *T. achaeae* to control the pest *T. absoluta* prove that the individuals of the parasitoid selected and reared according to the system described in this invention present numerous advantages against other species of natural enemies previously used in other countries. Thus, for commercial producers of biological control agents, *T. achaeae* is an effective natural enemy, preferably to control *T. absoluta* and also to control other pest noctuidae of small size, such as *H. armigera* and *C. chalcites.*

### References

Branco, M.C. & Franca, F.H. (1993). Susceptibility of three populations of Scrobipalpuloides absoluta (Lep.: Gelechiidae) to cartap. Hortic. bras. 11: 32-34.
Brotodjojo, R.R.R. & Walter, G.H. (2006). Oviposition and reproductive performance of a generalist parasitoid (Trichogramma pretiosum) exposed to host species that differ in their physical characteristics. Biol. Control 39: 300-312.
Cabello, T., Gallego, J.R., Vila, E., Soler, A., del Pino, M., Carnero, A., Hernández, E. & Polaszek, A. (2009). Biological control of the South American Tomato Pinworm, Tuta absoluta (Lep.: Gelechiidae), with releases of Trichogramma achaeae (Hym.: Trichogrammatidae) on tomato greenhouse of Spain. IOBCwprs Bull. 49: 225-230.
Driesche, R.G. van, Hoddle, M.S. & Center, T.D. (2007). Control de plagas y malezas por enemigos naturales. USDA. Washington: 751 pp. EPPO (2005). Tuta absoluta.OEPP/EPPO Bull., 35:434-435.
EPPO (2008). EPPO Reporting Service n° 1, 6 and 8.
Huber, J.T. , Darbyshire, S., Bissett, J. & Foottit, R.G. (2001). Taxonomy and Biological Control. In: Mason, P.G.; Huber, J.T. (Eds.). Biological Control Programmes in Canada, 1981-2000. C.A.B. Int. Wallingford: 14-22.
Lucas, A., Monserrat, A. & Soler, A. (2009). Incidencia de plagas y enfermedades en las Comunidades Autónomas en 2008: Murcia. Phytoma España 2007: 45-52.
Schlinger, E.I. & Doutt, R.L. (1984). La sistemática en relación con el control biológico. In: DeBach, P. (Ed.). Control biológico de las plagas de insectos y malas hierbas. CECSA. México: 283-317.
Urbaneja, A., Monton, H., Mollá, O. & Beitia, F. (2008). Suitability of the tomato borer Tuta absoluta as prey for Macrolophus pygmaeus and Nesidiocoris tenuis. Journal of Applied Entomology (publ. on line).
Urbaneja, A., Vercher, R., Navarro, V. & Garcia-Mari, F. & Porcuna, J.L. (2007). La polilla del tomate, Tuta absoluta. Phytoma-España, 194: 16-23.
Waterhouse, D.F. (1998). Biological Control of Insect Pests: Southeast Asian Prospects. Australian Centre for International Agricultural Research. Canberra: 548 pp.
Waterhouse, D.F. & Sands, D.P.A. (2001). Classical Biological Control of Arthropods in Australia. Australian Centre for International Agricultural Research. Canberra: 559 pp.

## Claims

1. Process for rearing the parasitoid *Trichogramma achaeae* on the moth *Ephestia kuehniella* or *Sitotroga cerealella* as factitious host.

2. Rearing process according to claim 1, wherein the parasitoid *Trichogramma achaeae* is used as a composition of at least two different subspecies.

3. Rearing process according to claim 2, wherein the parasitoid *Trichogramma achaeae* is used as a composition of three different subspecies.

4. Rearing process according to any of the preceding claims, wherein the population of the factitious host is used in the form of UV sterilized eggs.

5. Rearing process according to any of the preceding claims, wherein the parasitoid *Trichogramma achaeae* is collected from areas with Mediterranean or subtropical climate during summer period.

6. Rearing process according to any of the preceding claims, wherein the species *Trichogramma achaeae* is mantained at 20-25°C, preferably 22°C, at a relative humidity of 65-85%.

7. Rearing process according to any of the preceding claims, wherein said process comprises a container holding the species *Trichogramma achaeae* suitable for the adequate development of the parasitoid, guaranteeing the exchange of oxygen and enabling the exit of the mobile life stages of the parasitoid surfaced from the eggs of its factitious host.

8. Rearing process according to any of the preceding claims, wherein said container is directly hung on the plants.

9. Use of the parasitoid *Trichogramma achaeae* for biological pest control in commercial crops.

10. Use according to claim 9, wherein the parasitoid *Trichogramma achaeae* is reared according to any of claims 1 - 8.

11. Use according to claim 9 or 10, wherein the crop pest is selected from gelechii lepidopterans, such as *Tuta absoluta,* and noctuidae such as *Helicoverpa armigera* and *Chrysodeixis chalcites.*

12. Use according to any of the preceding claims, wherein the commercial crop is selected from protected vegetable crops of glass greenhouses, plastic greenhouses or crops under mesh, and also outdoors crops.

13. Use according to any of the preceding claims, wherein the species *Trichogramma achaeae* is distributed on the crops adhered to an inert material suitable for direct sprinkling application.

14. A composition comprising: a viable population of the parasitoid *Trichogramma achaeae,* and the moth *Ephestia kuehniella* or *Sitotroga cerealella* as factitious host.
